# EUROPÄISCHE PATENTSCHRIFT

(11) **EP 1 583 761 B1**
(45) Veröffentlichungstag und Bekanntmachung des Hinweises auf die Patenterteilung: **23.02.2011**
(21) Anmeldenummer: 03814467.1
(22) Anmeldetag: 24.12.2003
(51) Int. Cl.: C07D 413/14, C07D 413/12, C07D 333/44

(54) **VERFAHREN ZUR HERSTELLUNG VON 5-CHLOR- i N /i -({5S)-2-OXO-3- 4-(3-OXO-4-MORPHOLINYL)-PHENYL -1,3-OXAZOLIDIN-5-YL&r cub;-METHYL)-2-THIOPHENCARBOXAMID**
METHOD FOR PRODUCING 5-CHLORO- i N /i -({5 i S /i )-2-OXO-3- 4-(3-OXO-4-MORPHOLINYL)-PHENYL -1,3-OXAZOLIDIN-5-YL&rc ub;-METHYL)-2-THIOPHENE CARBOXAMIDE
PROCEDE DE PRODUCTION DE 5-CHLORO-N-( (5S)-2-OXO-3- 4-(3-OXO-4-MORPHOLINYLE)-PHENYLE -1,3-OXAZOLIDINE-5-YLE -METHYLE)-2-THIOPHENE-CARBOXAMIDE

(30) Priorität: 07.01.2003 DE 10300111
(43) Veröffentlichungstag der Anmeldung: 12.10.2005
(73) Patentinhaber: Bayer Schering Pharma Aktiengesellschaft, 13353 Berlin (DE)
(72) Erfinder: THOMAS, Christian, R., 42113 Wuppertal (DE)
(86) Internationale Anmeldenummer: PCT/EP2003/014871
(87) Internationale Veröffentlichungsnummer: WO 2004/060887

(56) Entgegenhaltungen:
- WO-A-01/47919
- WO-A-03/000256
- DATABASE BEILSTEIN BEILSTEIN INSTITUTE FOR ORGANIC CHEMISTRY, FRANKFURT-MAIN, DE; XP002278203 Database accession no. 8822985 & J.ORG.CHEM, Bd. 67, Nr. 11, 2002, Seiten 3933-3936,

## Beschreibung

Die vorliegende Erfindung betrifft ein Verfahren zur Herstellung von 5-Chlor-*N-*({(5*S*)-2-oxo-3-[4-(3-oxo-4-morpholinyl)-phenyl]-1,3-oxazolidin-5-yl}-methyl)-2-thiophencarboxamid ausgehend von 5-Chlorthiophen-2-carbonylchlorid, (2S)-3-Amino-propan-1,2-diol und 4-(4-Aminophenyl)-3-morpholinon.

Die Verbindung 5-Chlor-*N*-({(5*S*)-2-oxo-3-[4-(3-oxo-4-morpholilyl)-phenyl]-1,3-oxazolidin-5-yl}methyl)-2-thiophencarboxamid ist aus WO-A O1/47919 bekannt und entspricht der Formel (I)

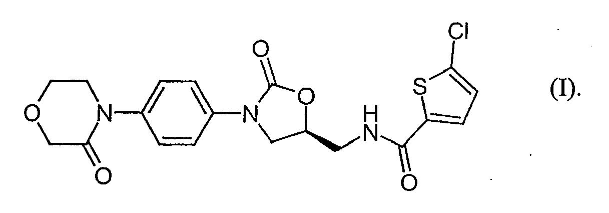

Die Verbindung der Formel (I) wirkt als Inhibitor des Blutgerinnungsfaktors Xa und kann als Mittel zur Prophylaxe und/oder Behandlung von thromboembolischen Erkrankungen insbesondere Herzinfarkt, Angina Pectoris (eingeschlossen instabile Angina), Reokhlusionen und Restenosen nach einer Angioplastie oder aortokoronarem Bypass, Hirnschlag, transitorische ischämische Attacken, periphere arterielle Verschlusskrankheiten, Lungenembolien oder tiefe venöse Thrombosen eingesetzt werden.

In WO-A 01/47919 ist auch eine Methode zur Herstellung der Verbindung der Formel (I) ausgehend von 2-[(2*S*)-2-Oxiranylmethyl]-1*H*-isoindol-1,3(2*H*)-dion (II), 4-(4-Aminophenyl)-3-morpholinon (III) und 5-Chlorthiophen-2-carbonylchlorid (IV) beschrieben:

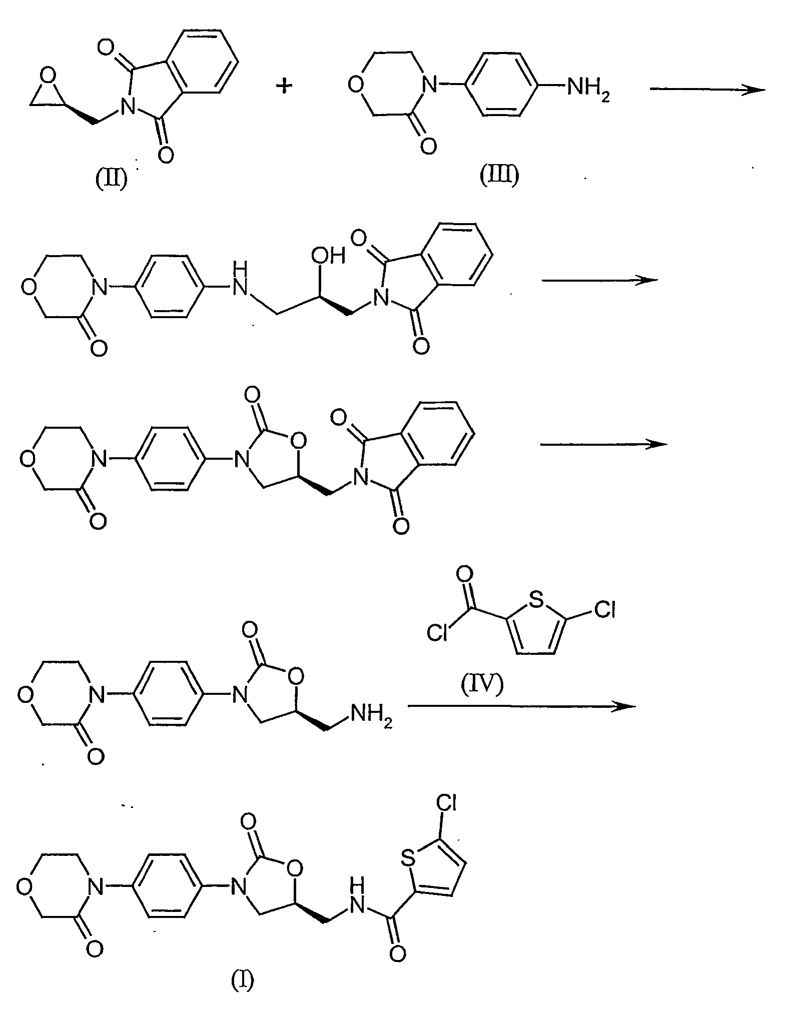

Das Epoxyphtalimid (II) wird dabei durch Umsetzung von (2S)-1-Chlorpropan-2,3-diol (V) mit Kaliumcarbonat über die Stufe des (*S*)-Glycidols (VI) und anschließende Mitsunobu-Reaktion mit Phtalimid hergestellt: 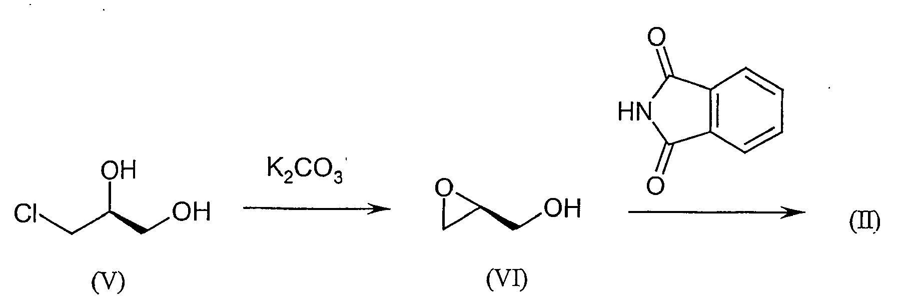 Das aus WO-A 01/47919 bekamte Verfahren weist verschiedene Nachteile auf, die sich besonders ungünstig bei der Herstellung der Verbindung der Formel (I) in technischem Maßstab auswirken:
So ist das Glycidol (VI) insbesondere in größeren Mengen polymerisationsempfindlich und damit nicht lagerstabil, darüber hinaus toxisch und potentiell carcinogen. Die Mitsunobu-Reaktion bei der Herstellung von Verbindung (II) ist technisch aufwendig, da zum einen bei größeren Ansätzen leicht Racemisierung auftritt. Zum anderen ist die Atomökonomie äußerst unbefriedigend, da in stöchiometrischen Mengen Triphenylphosphinoxid und Düsopropylazodicarboxylat-Hydrazid als Abfallstoffe erzeugt werden. Darüber hinaus wird das Stickstoffatom im Oxazolidinonring des Zielmoleküls (I) Phthalimid-geschützt eingeführt. Der Phthalsäurerest als Schutzgruppe muss aber im weiteren Verlauf der Synthese entfernt werden, was die Anzahl an Stufen erhöht und zusätzlichen Abfall bedeutet.

Daraus ergibt sich die Aufgabe der vorliegenden Erfindung, ein vereinfachtes Verfahren zur Herstellung der Verbindung der Formel (I) in großen Mengen bereitzustellen.

Überraschend wurde nun gefunden, dass man die Verbindung der Formel (I) in einer verkürzten Reaktionssequenz unter Verwendung von lagerstabilen und weniger toxischen Ausgangsstoffen ausgehend von 5-Chlorthiophen-2-carbonylchlorid (IV), (2*S*)-3-Amino-propan-1,2-diol Hydrochlorid (VII) und 4-(4-Aminophenyl)-3-morpholinon (III) in verbesserter Ausbeute herstellen kann. Hierbei wird auch die Verwendung von Schutzgruppen vermieden, was die Anzahl an Stufen reduziert und somit die Reaktionszeit verkürzt.

Im ersten Schritt des erfindungsgemäßen Verfahrens wird 5-Chlorthiophen-2-car-bonylchlorid (IV) 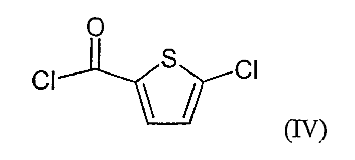 aus 5-Chlorthiophen-2-carbonsäure hergestellt.

Die Herstellung von Verbindung (IV) kann dabei unter den üblichen Reaktionsbedingungen für die Herstellung von Carbonsäurechloriden aus den entsprechenden Carbonsäuren erfolgen. Bevorzugt ist die Umsetzung von 5-Chlorthiophen-2-carbonsäure mit Thionylchlorid als Chlorierungsreagenz in Toluol als Lösungsmittel.

Im zweiten Schritt des erfindungsgemäßen Verfahrens wird 5-Chlorthiophen-2-carbonylchlorid (IV) mit (2*S*)-3-Amino-propan-1,2-diol Hydrochlorid (VII) 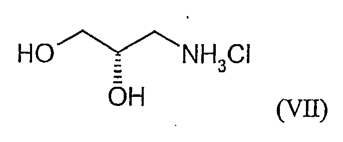 zu 5-Chlorthiophen-2-carbonsäure-((*S*)-2,3-dihydroxy-propyl)-amid (VIII) 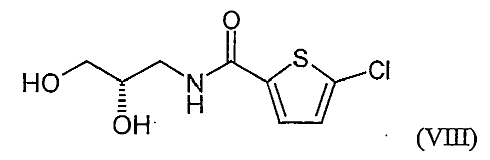 umgesetzt.

Die Reaktion (IV) + (VII) -> (VIII) kann unter den für die Knüpfung von Amidbindungen aus den entsprechenden Carbonsäurechloriden und Aminen üblichen Reaktionsbedingungen erfolgen. Bevorzugt ist ein Zweiphasensystem aus wässriger Natriumhydrogencarbonatlösung und 2-Methyltetrahydrofuran als organischem Lösungsmittel. (2*S*)-3-Amino-propan-1,2-diol wird als freie Base oder als Säureadditionssalz eingesetzt. Bevorzugt ist das Hydrochlorid (VII), das besser kristallisiert als die freie Base und deshalb gut handhabbar ist. Zur Erhöhung der Reaktionsausbeute wird gegebenenfalls entweder ein Überschuss an Amin verwendet oder es wird eine Hilfsbase zugesetzt. Der Zusatz von 1 bis 3, vorzugsweise 2, Äquivalenten einer Hilfsbase wie Natriumhydrogencarbonat ist bevorzugt. Die Reaktion erfolgt im allgemeinen in einem Temperaturbereich von 0°C bis 40°C, bevorzugt von 5°C bis 30°C.

Im dritten Schritt des erfmdungsgemäßen Verfahrens wird 5-Chlorthiophen-2-carbonsäure-((*S*)-2,3-dihydroxy-propyl)-amid (VIII) in 5-Chlorthiophen-2-carbonsäure-((*S*)-3-brom-2-hydroxy-propyl)-amid (IX) 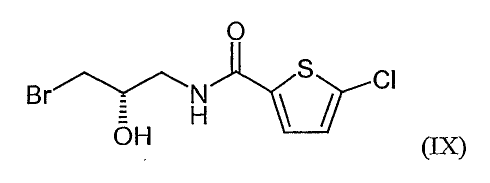 überführt.

Die Umsetzung (VIII) -> (IX) wird mit 1 bis 5 vorzugsweise 3 bis 5, insbesondere 4 Äquivalenten einer Lösung von Bromwasserstoffsäure in Essigsäure, gegebenenfalls in Gegenwart von Essigsäureanhydrid durchgeführt. Die Reaktionstemperatur liegt dabei zwischen 20°C und 80°C, vorzugsweise zwischen 60 und 65°C. Die zugesetzte Methanolmenge kann über einen breiten Bereich variiert werden, vorzugsweise werden 40 bis 80 Mol, insbesondere 50 bis 60 Mol, Methanol pro Mol (VIII) eingesetzt. Für die Aufarbeitung werden die Lösungsmittel abdestilliert, vorzugsweise im Vakuum. Der zurückbleibende Destillationssumpf wird gegebenenfalls vor der Filtration des Produktes noch neutralisiert.

Im vierten Schritt des erfindungsgemäßen Verfahrens wird Chlorthiophen-2-carbonsäure-((*S*)-3-brom-2-hydroxy-propyl)-amid (IX) mit 4-(4-Aminophenyl)-3-morpholinon (III) 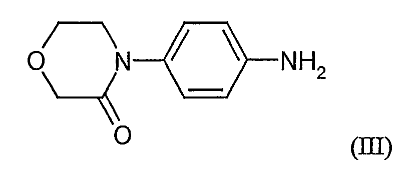 zu 5-Chlorthiophen-2-carbonsäure-{(*R*)-2-hydroxy-3-[4-(3-oxo-morpholin-4-yl)-phenylamino]-propyl}-amid (X) 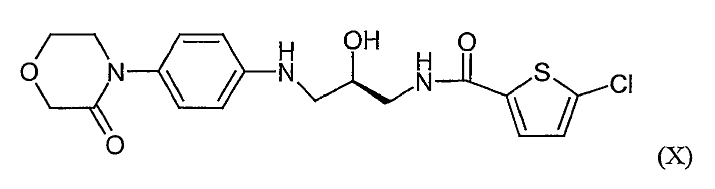 umgesetzt.

Das Lösungsmittel für die Reaktion (IX) + (III) -> (X) kann breit variiert werden, bevorzugt ist Toluol. Die Reaktionstemperatur liegt dabei zwischen 80°C und 200°C, bevorzugt ist ein Bereich zwischen 90°C und 110°C. Die Umsetzung erfolgt gegebenenfalls in Gegenwart einer Hilfsbase wie beispielsweise Triethylamin, Diisopropyethylamin oder Collidin, bevorzugt ist die Verwendung von Collidin. Die Stöchiometrie der Reaktion und die Reaktionszeit sind über einem breiten Bereich variierbar, bevorzugt ist ein Verhältnis von Verbindung (IX) zu Verbindung (III) zu Collidin von 1.2 zu 1.0 zu 1.0 und eine Reaktionszeit von 4 bis 8, insbesondere von 5 bis 6 Stunden.

Im fünften Schritt des erfindungsgemäßen Verfahrens wird 5-Chlorthiophen-2-carbonsäure- {(*R*)-2-hydroxy-3-[4-(3-oxo-morpholin-4-yl)-phenylamino]-propyl} - amid (X) mit Phosgen oder einem Phosgenäquivalent zu 5-Chlor-*N*-({(5*S*)-2-oxo-3-[4-(3-oxo-4-morpholinyl)-phenyl]-1,3-oxazolidin-5-yl}-methyl)-2-thiophencarboxamid (I) umgesetzt.

Bei der Umsetzung (X) -> (I) werden ein oder mehrere Äquivalente Phosgen oder Phosgenäquivalente in Gegenwart inerter Lösungsmittel oder Lösungsmittelgemische eingesetzt. Phosgenäquivalente sind beispielsweise Phosgenersatzstoffe wie Di- oder Triphosgen oder Kohlenmonoxidäquivalente wie beispielsweise N,N-Carbonyl-bisimidazol. Bevorzugt ist die Verwendung von 1 bis 2 Äquivalenten, insbesondere 1.1 bis 1.3 Äquivalenten, N,N-Carbonylbisimidazol in einem Lösungsmittelgemisch aus 1-Methyl-2-pyrrolidon und Toluol. Zur Reinigung des Produktes schließt sich gegebenenfalls eine Klärfiltration und/oder eine Umkristallisation an. Die Reaktion erfolgt im allgemeinen in einem Temperaturbereich von 20°C bis 150°C, bevorzugt von 30°C bis 110°C, insbesondere von 75°C bis 85°C.

Die einzelnen Stufen des erfindungsgemäßen Verfahrens können bei normalem, erhöhtem oder bei erniedrigtem Druck durchgeführt werden (z.B. von 0,5 bis 5 bar). Im allgemeinen arbeitet man bei Normaldruck.

Das folgende Schema fasst die Synthese zusammen:

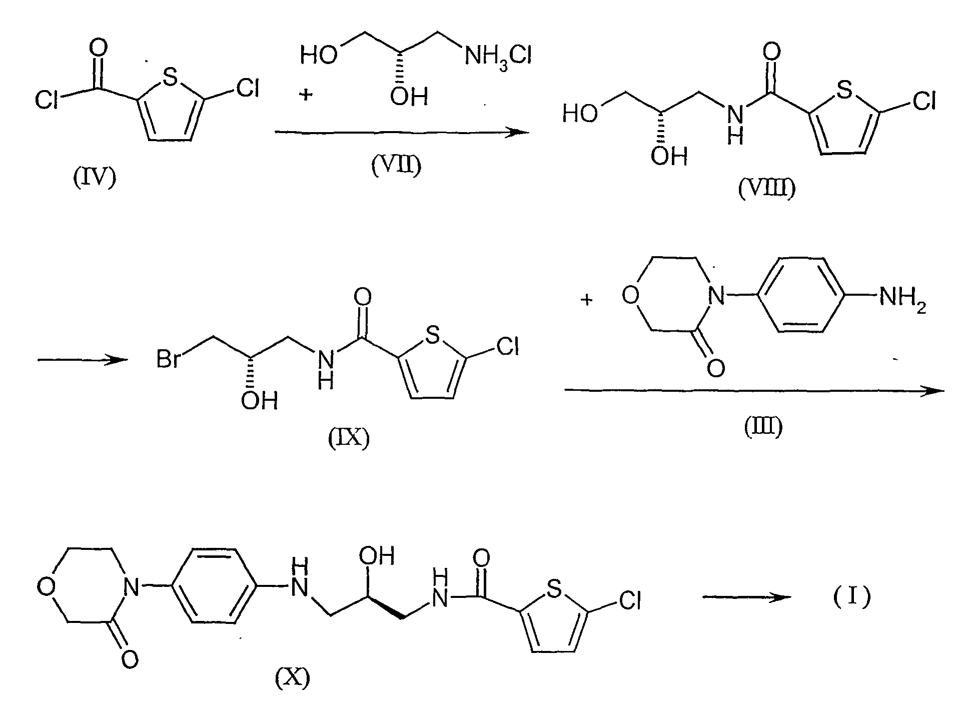

Die Erfindung wird nachstehend durch ein bevorzugtes Ausführungsbeispiel näher erläutert, auf welches sie jedoch nicht eingeschränkt ist. Soweit nicht anders angegeben, beziehen sich nachstehend alle Mengenangaben auf Gewichtsprozente.

### Synthese von 5-Chlor-N-({(5S)-2-oxo-3-[4-(3-oxo-4-morpholinyl)-phenyl]-1,3-oxazolidin-5-yl}-methyl)-2-thiophencarboxamid (I)

### 1. Schritt:

### 5-Chlorthiophen-2-carbonylchlorid (IV)

53,6 g 5-Chlorthiophen-2-carbonsäure (kommerziell erhältlich) werden in 344 g Toluol suspendiert und auf 80°C erwärmt. Bei dieser Temperatur werden 47,2 g Thionylchlorid über einen Zeitraum von 20 Minuten zugetropft, anschließend wird 30 Minuten bei 75 bis 80°C und dann zwei Stunden bei Rückflusstemperatur bis zur Beendigung der Gasentwicklung nachgeführt. Nach dem Abkühlen wird das Reaktionsgemisch bei 30 bis 35°C und einem Druck von 40 bis 48 mbar auf ein Volumen von ca. 200 ml eingeengt. Die so erhaltene Lösung des Säurechlorids in Toluol wird direkt in der nächsten Stufe umgesetzt.

### 2. Schritt:

### 5-Chlorthiophen-2-carbonsäure-((S)-2,3-dihydroxy-propyl)-amid (VIII)

461 g Natriumhydrogencarbonat und 350 g (2*S*)-3-Amino-propan-1,2-diol Hydrochlorid (VII) (kommerziell erhältlich) werden bei 13 bis 15°C in 2,1 Wasser vorgelegt und mit 950 ml 2-Methyltetrahydrofuran versetzt. Zu dieser Mischung werden unter Kühlung bei 15 bis 18°C 535,3 g 5-Chlorthiophen-2-carbonylchlorid (ca. 93 %ig) in 180 ml Toluol über einen Zeitraum von zwei Stunden zugetropft. Zur Aufarbeitung werden die Phasen getrennt und die organische Phase wird in mehreren Schritten mit insgesamt 1,5 1 Toluol versetzt. Das ausgefallene Produkt wird abgesaugt, mit Essigsäureethylester gewaschen und getrocknet.
Ausbeute: 593,8 g; entspricht 91,8 % der Theorie.
Schmelzpunkt: 114 bis 114,5°C

### 3. Schritt:

### 5-Chlorthiophen-2-carbonsäure-((S)-3-brom-2-hydroxy-propyl)-amid (IX)

Zu einer Suspension von 100 g 5-Chlorthiophen-2-carbonsäure-((*S*)-2,3-dihydroxypropyl)-amid (VTII) in 250 ml Eisessig werden bei 21 bis 26°C über einen Zeitraum von 30 Minuten 301,7 ml einer 33 %ige Lösung von Bromwasserstoffsäure in Essigsäure zugegeben. Anschließend werden 40 ml Essigsäureanhydrid zugegeben und der Reaktionsansatz wird drei Stunden bei 60 bis 65°C gerührt. Bei 20 bis 25°C werden dann über einen Zeitraum von 30 Minuten 960 ml Methanol zugegeben. Das Reaktionsgemisch wird 2,5 Stunden unter Rückfluss und dann über Nacht bei 20 bis 25°C gerührt. Zur Aufarbeitung werden die Lösungsmittel im Vakuum bei ca. 95 mbar abdestilliert. Die zurückbleibende Suspension wird mit 50 ml 1-Butanol und 350 ml Wasser versetzt. Das ausgefallene Produkt wird abgesaugt, mit Wasser gewaschen und getrocknet.
Ausbeute: 89,8 g; entspricht 70,9 % der Theorie.
Schmelzpunkt: 120°C

### 4. Schritt:

### 5-Chlorthiophen-2-carbonsäure-{(R)-2-hydroxy-3-[4-(3-oxo-morpholin-4-yl)-phenylamino]-propyl}-amid (X)

55 g 5-Chlorthiophen-2-carbonsäure-((*S*)-3-brom-2-hydroxy-propyl)-amid (IX) und 29,4 g 4-(4-Aminophenyl)-3-morpholinon (III) (eine Herstellmethode ist beispielsweise in WO-A 01/47919 auf den Seiten 55 bis 57 beschrieben) werden bei 20 bis 25°C in 500 ml Toluol suspendiert und mit 18,5 g Collidin und 10 ml Ethanol versetzt. Der Reaktionsansatz wird 6 Stunden auf 103 bis 105°C erhitzt und dann in der Hitze mit 50 ml 1-Butanol versetzt. Nach Abkühlen auf 30°C wird das ausgefallene Reaktionsprodukt abgesaugt, mit Toluol und Wasser gewaschen und getrocknet.
Ausbeute: 42,0 g; entspricht 61,8 % der Theorie.
Schmelzpunkt: 198,5°C

### 5. Schritt:

### 5-Chlor-N-({(5S)-2-oxo-3-[4-(3-oxo-4-morpholinyl)-phenyl]-1,3-oxazolidin-5-yl}methyl)-2-thiophencarboxamid (I)

25 g 5-Chlorthiophen-2-carbonsäure-{(*R*)-2-hydroxy-3-[4-(3-oxo-morpholin-4-yl)-phenylamino]-propyl}-amid (X) werden bei 20 bis 25°C in 250 ml Toluol suspendiert und mit 37,5 ml 1-Methyl-2-pyrrolidon und 11,9 g N,N-Carbonyldiimidazol versetzt. Der Reaktionsansatz wird 20 Minuten auf 80 bis 83°C und anschließend eine Stunde auf 115°C erhitzt. Nach Abkühlen auf 20°C wird das ausgefallene Reaktionsprodukt abgesaugt, zweimal mit je 25 ml Wasser gewaschen und bei 60°C im Vakuum getrocknet.
Ausbeute: 23,7 g; entspricht 91,5 % der Theorie.
Schmelzpunkt: 230°C

## Patentansprüche

1. Verfahren zur Herstellung von 5-Chlor-*N*-({(5*S*)-2-oxo-3-[4-(3-oxo-4-morpholinyl)-phenyl]-1,3-oxazolidin-5-yl}-methyl)-2-thiophencarboxamid der Formel (I), **dadurch gekennzeichnet, dass** man
in einem ersten Schritt durch Chlorierung von 5-Chlorthiophen-2-carbonsäure 5-Chlorthiophen-2-carbonylchlorid (IV) herstellt, dieses dann
in einem zweiten Schritt mit (2*S*)-3-Amino-propan-1,2-diol Hydrochlorid (VII) zu 5-Chlorthiophen-2-carbonsäure-((*S*)-2,3-dihydroxy-propyl)-amid (VIII) umsetzt, dieses dann
in einem dritten Schritt in 5-Chlorthiophen-2-carbonsäure-((*S*)-3-brom-2-hydroxy-propyl)-amid (IX) überführt, dieses dann
in einem vierten Schritt durch Umsetzung mit 4-(4-Aminophenyl)-3-morpholinon (III) in 5-Chlorthiophen-2-carbonsäure-{(*R*)-2-hydroxy-3-[4-(3-oxo-morpholin-4-yl)-phenylamino]-propyl}-amid (X) überführt und dieses dann
in einem fünften Schritt mit Phosgen oder einem Phosgenäquivalent umsetzt.

2. Verfahren zur Herstellung von 5-Chlorthiophen-2-carbonsäure-{(*R*)-2-hydroxy-3-[4-(3-oxo-morpholin-4-yl)-phenylamino]-propyl}-amid (X), **dadurch gekennzeichnet, dass** man 5-Chlorthiophen-2-carbonsäure-((*S*)-3-brom-2-hydroxy-propyl)-amid (IX) mit 4-(4-Aminophenyl)-3-morpholinon (III) umsetzt.

3. Verfahren zur Herstellung von 5-Chlor-*N*-({(5*S*)-2-oxo-3-[4-(3-oxo-4-morpholinyl)-phenyl]-1,3-oxazolidin-5-yl}-methyl)-2-thiophencarboxamid der Formel (I), **dadurch gekennzeichnet, dass** man 5-Chlorthiophen-2-carbonsäure-{(*R*)-2-hydroxy-3-[4-(3-oxo-morpholin- 4-yl)-phenylamino]-propyl}-amid (X) durch Umsetzung von 5-Chlor- thiophen-2-carbonsäure-((*S*)-3-brom-2-hydroxy-propyl)-amid (IX) mit 4-(4- Aminophenyl)-3-morpholinon (III) herstellt und anschielßend das 5-Chlorthiophen-2-carbonsäure- {(*R*)-2-hydroxy-3-[4-(3-oxo-morpholin-4-yl)-phenylamino]-propyl}-amid (X) mit Phosgen oder einem Phosgenäquivalent umsetzt.

4. Verfahren nach Anspruch 3, **dadurch gekennzeichnet, dass** das Phosgenäquivalent N,N-Carbonyldiimidazol ist.

5. Verfahren nach Anspruch 4, **dadurch gekennzeichnet, dass** 1.1 bis 1.3 Äquivalente N,N-Carbonyldiimidazol eingesetzt werden.

6. Verfahren nach einem der Ansprüche 3 bis 5, **dadurch gekennzeichnet, dass** die Umsetzung in einem Lösungsmittelgemisch aus 1-Methyl-2-pyrrolidon und Toluol stattfindet.

7. Verfahren nach einem der Ansprüche 3 bis 6, **dadurch gekennzeichnet, dass** das 5-Chlorthiophen-2-carbonsäure-((*S*)-3-brom-2-hydroxy-propyl)-amid (IX) durch Umsetzung von 5-Chlorthiophen-2-carbonsäure-((*S*)-2,3-dihydroxypropyl)-amid (VIII) hergestellt wird.

8. Verfahren nach Anspruch 7, **dadurch gekennzeichnet, dass** das 5-Chlorthiophen-2-carbonsäure-((*S*)-2,3-dihydroxy-propyl)-amid (VIII) durch Umsetzung von 5-Chlorthiophen-2-carbonylchlorid (IV) mit (2*S*)-3-Amino-propan-1,2-diol Hydrochlorid (VII) hergestellt wird.

9. Chlorthiophen-2-carbonsäure-((*S*)-3-brom-2-hydroxy-propyl)-amid der Formel (IX)

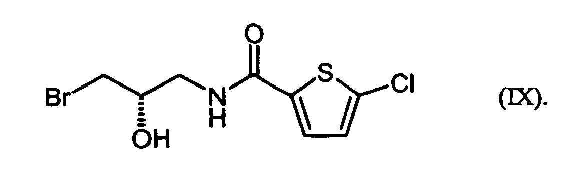

## Claims

1. Process for preparing 5-chloro-*N*-({(5*S*)-2-oxo-3-[4-(3-oxo-4-morpholinyl)phenyl]-1,3-oxazolidin-5-yl}methyl)-2-thiophenecarboxamide of the formula (I), **characterized in that**
5-chlorothiophene-2-carbonyl chloride (IV) is prepared in a first step by chlorinating 5-chlorothiophene-2-carboxylic acid, and is then
reacted in a second step with (2*S*)-3-aminopropane-1,2-diol hydrochloride (VII) to give N-((*S*)-2,3-dihydroxypropyl)-5-chlorothiophene-2-carboxamide (VIII) which is then
converted in a third step to N-((*S*)-3-bromo-2-hydroxypropyl)-5-chlorothiophene-2-carboxamide (IX) which is then
converted in a fourth step by reacting with 4-(4-aminophenyl)-3-morpholinone (III) to N-{(*R*)-2-hydroxy-3-[4-(3-oxomorpholin-4-yl)phenylamino]propyl}-5-chlorothiophene-2-carboxamide (X) which is then
reacted in a fifth step with phosgene or a phosgene equivalent.

2. Process for preparing N-{(*R*)-2-hydroxy-3-[4-(3-oxomorpholin-4-yl)phenylamino]propyl}-5-chlorothiophene-2-carboxamide (X), **characterized in that** N-((*S*)-3-bromo-2-hydroxypropyl)-5-chlorothiophene-2-carboxamide (IX) is reacted with 4-(4-aminophenyl)-3-morpholinone (III).

3. Process for preparing 5-chloro-*N*-({(5*S*)-2-oxo-3-[4-(3-oxo-4-morpholinyl)phenyl]-1,3-oxazolidin-5-yl}methyl)-2-thiophenecarboxamide of the formula (I), **characterized in that** N-{(*R*)-2-hydroxy-3-[4-(3-oxomorpholin-4-yl)phenylamino]propyl}-5-chlorothiophene-2-carboxamide (X) is prepared by reacting N-((*S*)-3-bromo-2-hydroxypropyl)-5-chlorothiophene-2-carboxamide (IX) with 4-(4-aminophenyl)-3-morpholinone (III), and then the N-{(*R*)-2-hydroxy-3-[4-(3-oxomorpholin-4-yl)phenylamino]propyl}-5-chlorothiophene-2-carboxamide (X) is reacted with phosgene or a phosgene equivalent.

4. Process according to Claim 3, **characterized in that** the phosgene equivalent is N,N-carbonyldiimidazole.

5. Process according to Claim 4, **characterized in that** from 1.1 to 1.3 equivalents of N,N-carbonyldiimidazole are used.

6. Process according to one of Claims 3 to 5, **characterized in that** the reaction takes place in a solvent mixture of 1-methyl-2-pyrrolidone and toluene.

7. Process according to one of Claims 3 to 6, **characterized in that** the N-((*S*)-3-bromo-2-hydroxypropyl)-5-chlorothiophene-2-carboxamide (IX) is prepared by converting N-((*S*)-2,3-dihydroxypropyl)-5-chlorothiophene-2-carboxamide (VIII).

8. Process according to Claim 7, **characterized in that** the N-((*S*)-2,3-dihydroxypropyl)-5-chlorothiophene-2-carboxamide (VIII) is prepared by reacting 5-chlorothiophene-2-carbonyl chloride (IV) with (2*S*)-3-aminopropane-1,2-diol hydrochloride (VII).

9. N-((*S*)-3-Bromo-2-hydroxypropyl)chlorothiophene-2-carboxamide of the formula (IX)

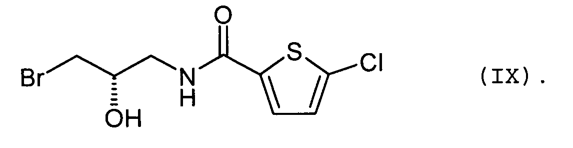

## Revendications

1. Procédé de fabrication de 5-chloro-N-({(5S)-2-oxo-3-[4-(3-oxo-4-morpholinyl)-phényl]-1,3-oxazolidin-5-yl}-méthyl)-2-thiophènecarboxamide de formule (I), **caractérisé en ce que**
lors d'une première étape, du chlorure de 5-chlorothiophène-2-carbonyle (IV) est fabriqué par chloration d'acide 5-chlorothiophène-2-carboxylique, puis
lors d'une deuxième étape, celui-ci est mis en réaction avec de l'hydrochlorure de (2S)-3-amino-propane-1,2-diol (VII) pour former du ((S)-2,3-dihydroxy-propyl)-amide de l'acide 5-chlorothiophène-2-carboxylique (VIII), puis
lors d'une troisième étape, celui-ci est transformé en ((S)-3-bromo-2-hydroxy-propyl)-amide de l'acide 5-chlorothiophène-2-carboxylique (IX), puis lors d'une quatrième étape, celui-ci est transformé en {(R)-2-hydroxy-3-[4-(3-oxo-morpholin-4-yl)-phénylamino]-propyl}-amide de l'acide 5-chlorothiophène-2-carboxylique (X) par réaction avec de la 4-(4-aminophényl)-3-morpholinone (III), puis lors d'une cinquième étape, celui-ci est mis en réaction avec du phosgène ou un équivalent de phosgène.

2. Procédé de fabrication de {(R)-2-hydroxy-3-[4-(3-oxo-morpholin-4-yl)-phénylamino]-propyl}-amide de l'acide 5-chlorothiophène-2-carboxylique (X), **caractérisé en ce que** du ((S)-3-bromo-2-hydroxy-propyl)-amide de l'acide 5-chlorothiophène-2-carboxylique (IX) est mis en réaction avec de la 4-(4-aminophényl)-3-morpholinone (III).

3. Procédé de fabrication de 5-chloro-N-({(5S)-2-oxo-3-[4-(3-oxo-4-morpholinyl)-phényl]-1,3-oxazolidin-5-yl}-méthyl)-2-thiophènecarboxamide de formule (I), **caractérisé en ce que** du {(R)-2-hydroxy-3-[4-(3-oxo-morpholin-4-yl)-phénylamino]-propyl}-amide de l'acide 5-chlorothiophène-2-carboxylique (X) est fabriqué par mise en réaction de ((S)-3-bromo-2-hydroxy-propyl)-amide de l'acide 5-chlorothiophène-2-carboxylique (IX) avec de la 4-(4-aminophényl)-3-morpholinone (III), puis le {(R)-2-hydroxy-3-[4-(3-oxo-morpholin-4-yl)-phénylamino]-propyl}-amide de l'acide 5-chlorothiophène-2-carboxylique (X) est mis en réaction avec du phosgène ou un équivalent de phosgène.

4. Procédé selon la revendication 3, **caractérisé en ce que** l'équivalent de phosgène est le N,N-carbonyldiimidazol.

5. Procédé selon la revendication 4, **caractérisé en ce que** 1,1 à 1,3 équivalents de N,N-carbonyldiimidazol sont utilisés.

6. Procédé selon l'une quelconque des revendications 3 à 5, **caractérisé en ce que** la réaction a lieu dans un mélange de solvants de 1-méthyl-2-pyrrolidone et de toluène.

7. Procédé selon l'une quelconque des revendications 3 à 6, **caractérisé en ce que** le ((S)-3-bromo-2-hydroxy-propyl)-amide de l'acide 5-chlorothiophène-2-carboxylique (IX) est fabriqué par réaction de ((S)-2,3-dihydroxy-propyl)-amide de l'acide 5-chlorothiophène-2-carboxylique (VIII).

8. Procédé selon la revendication 7, **caractérisé en ce que** le ((S)-2,3-dihydroxy-propyl)-amide de l'acide 5-chlorothiophène-2-carboxylique (VIII) est fabriqué par réaction de chlorure de 5-chlorothiophène-2-carbonyle (IV) avec de l'hydrochlorure de (2S)-3-amino-propane-1,2-diol (VII).

9. ((S)-3-bromo-2-hydroxy-propyl)-amide de l'acide chlorothiophène-2-carboxylique de formule (IX)
